# EUROPÄISCHE PATENTSCHRIFT

(11) **EP 1 614 630 B1**
(45) Veröffentlichungstag und Bekanntmachung des Hinweises auf die Patenterteilung: **02.05.2007**
(21) Anmeldenummer: 05011277.0
(22) Anmeldetag: 25.05.2005
(51) Int. Cl.: B65B 55/08, B67C 7/00

(54) **Verfahren sowie Vorrichtung zum Sterilisieren von Behältern mit UV-Strahlung**
Method and device for sterilizing containers with UV-rays
Méthode et dispositif pour stériliser des récipients avec des UV-rayons

(30) Priorität: 07.07.2004 DE 102004032861
(43) Veröffentlichungstag der Anmeldung: 11.01.2006
(73) Patentinhaber: KHS AG, 44143 Dortmund (DE)
(72) Erfinder: Till, Volker, 65719 Hofheim/Taunus (DE)

(56) Entgegenhaltungen:
- EP-A- 0 579 679
- EP-A- 1 046 585
- WO-A-02/36437
- DE-A1- 4 407 183
- US-A- 2 175 682
- US-A- 2 384 778
- US-A- 5 768 853

## Beschreibung

Die Erfindung bezieht sich auf ein Verfahren gemäß Patentanspruch 1. Weiterhin bezieht sich die Erfindung auf eine Vorrichtung gemäß Oberbegriff 7.

Das Sterilisieren von Behältern, speziell von Behältern und/oder Verpackungen für keimanfälliges Füllgut, beispielsweise für Getränke, Kosmetika usw. mit UV-Strahlung ist grundsätzlich bekannt (DE 44 07 183 A1, US 2,384,778 sowie WO 02/36437).

Allen bekannten Vorrichtungen und Verfahren zur UV-Sterilisation ist u.a. gemeinsam, dass für die Abstrahlung der UV-Strahlung bei der Innensterilisation von Behältern die UV-Quelle in den jeweiligen Behälter durch die Behälteröffnung eingeführt wird. Nachteilig ist hier zumindest, dass bei der Gestaltung der UV-Quelle u.a. auch die Art und Form der zu sterilisierenden Behälter berücksichtigt werden müssen, und dabei insbesondere der Öffnungsquerschnitt der Behälter, die konstruktive Ausbildung der UV-Quelle also nicht nur nach Gesichtspunkten einer möglichst optimalen Strahlungserzeugung und eines möglichst guten Wirkungsgrades vorgenommen werden kann.

Bekannt ist weiterhin auch ein Verfahren, bei dem zur Innensterilisation von Behältern deren Innenfläche mit einer in einem UV-Laser außerhalb des jeweiligen Behälters erzeugte UV-Strahlung und zugleich mit einer ebenfalls in einem Laser erzeugten Infrarotstrahlung behandelt wird (EP 0 579 679 B1). Das Einbringen der Strahlung in den jeweiligen Behälter erfolgt über Reflektoren.

Aufgabe der Erfindung ist es, ein Verfahren aufzuzeigen welches eine Innensterilisation von Behältern und/oder Verpackungen mit UV-Strahlung in besonders einfacher und gefahrloser Weise ermöglicht. Zur Lösung dieser Aufgabe ist ein Verfahren entsprechend dem Patentanspruch 1 ausgebildet. Eine Vorrichtung zum Durchführen des Verfahrens ist Gegenstand des Patentanspruches 7.

Die Besonderheit der Erfindung besteht darin, dass die Abstrahlung der UV-Strahlung, die von einer UV-Quelle außerhalb des jeweiligen Behälters erzeugt wird, im Inneren des Behälters durch ein in den Behälter über die Behälteröffnung eingeführtes flexibles Lichtleiterelement erfolgt. "Behälter" im Sinne der Erfindung sind u.a. Flaschen, aber auch aus Flachmaterial hergestellte Verpackungen z.B. für Getränke und andere flüssige Lebensmittel.

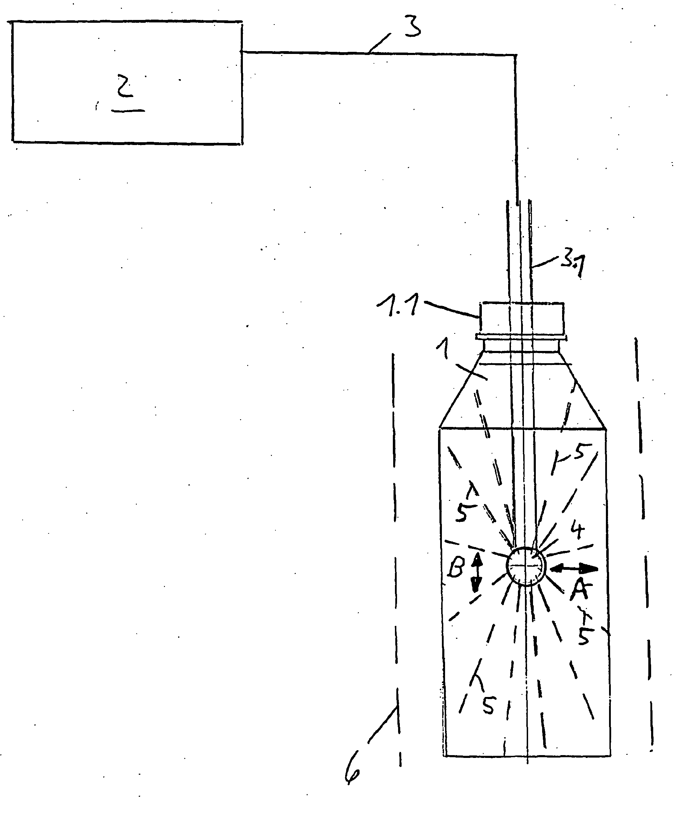

Weiterbildungen der Erfindung sind Gegenstand der Unteransprüche. Die Erfindung wird im Folgenden anhand der Figur an einem Ausführungsbeispiel erläutert.

In der Figur ist 1 eine Verpackung bzw. ein Behälter für ein keimanfälliges flüssiges oder pastöses Füllgut, beispielsweise für ein Getränk.

Vor dem Füllen mit dem Füllgut erfolgt eine Innensterilisation des Behälters 1 mittels UV-Strahlung in einem Wellenlängenbereich (z.B. 254 nm), der eine optimale Abtötung der auf der Behälterinnenfläche befindlichen Keime bewirkt. Die UV-Strahlung wird durch eine geeignete Strahlungsquelle 2 , beispielsweise eine Quecksilberdampfentladungslampe, einen UV-Laser und dergleichen mit ausreichend hoher Leistung erzeugt. Die Strahlungsquelle 2 ist außerhalb des jeweiligen Behälters 1 vorgesehen. Die UV-Strahlung wird in den Behälter über ein flexibles Lichtleiterelement 3 eingeleitet. Hierfür ist das Lichtleiterelement 3 mit einem Abschnitt 3.1 durch die Mündungsöffnung 1.1 in den Behälterinnenraum eingeführt und dort mit einem optischen Abstrahlelement 4 versehen, welches eine räumliche, d.h. z.B. kugelförmige Abstrahlung der UV-Strahlung mit einem Öffnungswinkel von 360° oder nahezu 360° in jeder Raumebene ermöglicht, so dass entsprechend den Wirklinien oder Pfeilen 5 die gesamte Innenfläche des Behälters 1 von der UV-Strahlung erfasst wird. Um beispielsweise bei einer speziellen Ausbildung und/oder Form des Behälters 1 durch Schattenbildung von der UV-Strahlung nicht erfasste Bereiche an der Behälterinnenfläche zu vermeiden, besteht die Möglichkeit, das in den Behälter 1 eingeführte Ende des Lichtleiterelementes 3 relativ zum Behälter 1 zu bewegen, wie dies mit den Doppelpfeilen A und B in der Figur angedeutet ist. Diese Bewegung ist bei flexibler Ausbildung des Lichtleiterelementes problemlos möglich.

Ebenfalls besteht die Möglichkeit, das Abstrahlelement 4 derart auszubilden, dass die Strahlungsrichtung während des Sterilisationsvorganges gesteuert verändert wird, wobei die UV-Strahlung vorzugsweise nicht kugelförmig sondern beispielsweise aus einer über den gesamten Umfang des Abstrahlelement 4 reichenden ringförmigen Abstrahlöffnung radial abgegeben wird, wobei der Austrittswinkel der Strahlung gesteuert verändert werden kann. Zur Realisierung dieser Funktion sind z.B. durch Piezoelemente bewegte Kleinstspiegel besonders geeignet.

Eine weitere, überaus vorteilhafte Weiterbildung der vorliegenden Erfindung sieht vor, das Abstrahlelement 4 derart auszubilden, dass die Intensität der kugelförmig abgegebenen UV-Strahlung über die Kugeloberfläche betrachtet nicht gleichmäßig sondern unterschiedlich ist. Durch diese Vorgehensweise ergibt sich der Vorteil, dass bei unveränderter oder sogar verminderter Leistung der UV-Quelle kritische Bereiche des Behälters besonders intensiv bestrahlt werden können. Um die Strahlungsintensität über die Oberfläche betrachtet ungleichmäßig auszubilden bietet es sich z.B. an, die Austrittsöffnungen der Strahlung nicht regelmäßig nach einem Muster, sondern gemäß der Intensitätsanforderungen anzuordnen.

Das Lichtleiterelement 3 besteht vorzugsweise aus einem Bündel von Lichtleiterfasern, wobei diese z.B. aus Glas oder einem anderen, für Lichtleiter geeignetem Material bestehen können.

In der praktischen Ausführung erfolgt die UV-Innensterilisation der Behälter 1 in einem Sterilisator, der in einer Produktionslinie einer dortigen Füllmaschine vorausgeht und der beispielsweise an einem um eine vertikale Maschinenachse umlaufend angetriebenen Rotor eine Vielzahl von Behälteraufnahmen 6 bildet, denen jeweils an einer Behälteraufgabe die zu sterilisierende Behälter 1 zugeführt werden, die dann an einer Behälterabgabe dem Rotor entnommen und an die nachfolgende Füllmaschine gefördert werden. Jede Aufnahme 6 weist dabei das mit der Optik 4 versehene Lichtleiterende 3.1 auf, welches dann für den Sterilisationsvorgang gesteuert in den Innenraum des Behälters 1 eingebracht und nach der Behandlung mit der UV-Strahlung aus dem betreffenden Behälter 1 wieder entfernt wird, und zwar beispielsweise durch entsprechendes Anheben und Absenken der Behälter 1. Jeder Aufnahme 6 ist z.B. eine eigenständige UV-Quelle 2 zugeordnet. Grundsätzlich besteht auch die Möglichkeit für sämtliche Aufnahmen oder jeweils für eine Gruppe von mehreren Aufnahmen eine gemeinsame UV-Quelle 2 vorzusehen.

Die erfindungsgemäße Ausbildung hat zahlreiche Vorteile. Durch das flexible Lichtleiterelement 3 ist, wie bereits erwähnt, eine Relativbewegung zwischen dem das Abstrahlelement 4 aufweisenden Ende und dem Behälter 1 zur Vermeidung von durch die UV-Strahlung nicht erfassten Bereichen möglich. Durch die flexible Ausbildung des Lichtleiterelementes 3 ist es weiter auch möglich, dieses innerhalb der Maschine (Sterilisator) in optimaler Weise anzuordnen, zu verlegen und zu montieren. Ein weiterer Vorteil besteht darin, dass das flexible Lichtleiterelement auch mit geringem mechanischen Aufwand den Bewegungen des jeweiligen Behälters folgen kann. Die UV-Strahlung ist im Lichtleiterelement zuverlässig geführt und eine die Gefahr von Verletzungen vermeidende Abschirmung ist problemlos möglich.

Da das Lichtleiterelement 3 mit geringem Außendurchmesser realisierbar ist, ist dieses Element auch in Behälter einführbar, die nur einen kleinen Durchmesser an der Behältermündung aufweisen. Weiterhin kann die jeweilige UV-Quelle 2 durch die erfindungsgemäße Ausbildung eine nahezu beliebige Gestalt aufweisen, d.h. die Konstruktion dieser Strahlungsquelle kann beispieisweise dem Gesichtspunkt einer möglichst optimalen Erzeugung der UV-Strahlung bei möglichst großem Wirkungsgrad folgend gewählt werden, und zwar völlig unabhängig von der speziellen Art und/oder Formgebung der zu sterilisierenden Behälter 1.

Die Erfindung eignet sich für die Innensterilisation von Behältern jeglicher Art, insbesondere auch für die Innensterilisation von aus Kunststoff und/oder aus Flachmaterial hergestellten Behältern oder Verpackungen.

### Bezugszeichenliste

- 1: Behälter
- 1.1: Behältermündung
- 2: UV-Quelle
- 3: Lichtleiterelement
- 3.1: Abschnitt des Lichtleiterelementes
- 4: Optisches Element zur räumlichen Abstrahlung der UV-Strahlung
- 5: UV-Strahlung
- 6: Bearbeitungsposition

- Pfeil A, Pfeil B: Relativbewegung zwischen dem Lichtleiter 3 bzw. dem Abstrahlelement 4 und dem Behälter 1

## Patentansprüche

1. Verfahren zum Sterilisieren von Behältern, bei dem die Behälterinnenfläche mit einer im Inneren des Behälters abgestrahlten UV-Strahlung einer UV-Quelle (2) behandelt wird, **dadurch gekennzeichnet,**
**dass** die UV-Strahlung der vollständig außerhalb des Behälters (1) befindlichen UV-Quelle (2) über ein durch eine Behältermündung (1.1) in den Behälterinnenraum eingeführtes Lichtleiterelement (3) abgestrahlt wird.

2. Verfahren nach Anspruch 1, **gekennzeichnet durch** die Verwendung eines flexiblen Lichtleiterelementes (3).

3. Verfahren nach Anspruch 1 oder 2, **gekennzeichnet durch** die Verwendung eines Lichtleiterelementes (3), welches an seinem in den jeweiligen Behälterinnenraum eingeführten Ende für eine Abstrahlung der UV-Strahlung (5) über einen räumlichen Öffnungswinkel von 360° oder annähernd 360° in jeder Raumebene ausgebildet ist.

4. Verfahren nach Anspruch 1 oder 2, **gekennzeichnet durch** die Verwendung eines Lichtleiterelementes (3), welches an seinem in den jeweiligen Behälterinnenraum eingeführten Ende für eine ringförmige Abstrahlung der UV-Strahlung (5) über einen räumlichen Öffnungswinkel von 360° ausgebildet ist, wobei der Austrittswinkel der UV-Strahlung (5) gesteuert veränderbar ist.

5. Vorrichtung zum Sterilisieren von Behältern (1) mit UV-Strahlung, mit einer UV-Strahlungsquelle (2), deren Strahlung im Inneren des Behälters abgestrahlt wird, **dadurch gekennzeichnet, dass** der Ausgang der UV-Quelle (2) mit mindestens einem Lichtleiterelement (3) verbunden ist, welches mit einem der UV-Quelle (2) entfernt liegenden Ende (3.1) durch die Behältermündung (1.1) in den jeweiligen Behälter zum Abstrahlen der UV-Strahlung einführbar ist.

6. Vorrichtung nach Anspruch 5, **dadurch gekennzeichnet, dass** das Lichtleiterelement (3) an dem Ende für eine Abstrahlung der UV-Strahlung (5) über einen räumlichen Öffnungswinkel von 360° oder nahezu 360° in jeder Raumebene ausgebildet ist.

7. Vorrichtung nach Anspruch 5, **dadurch gekennzeichnet, dass** das Lichtleiterelement (3) an dem Ende für eine radiale Abstrahlung der UV-Strahlung (5) über einen räumlichen Öffnungswinkel von 360° ausgebildet ist, wobei Mittel angeordnet sind, welche den Austrittswinkel der Strahlung gesteuert verändern.

8. Vorrichtung nach Anspruch 5, 6 oder 7, **dadurch gekennzeichnet, dass** das Lichtleiterelement (3) flexibel ist.

9. Vorrichtung nach einem der vorhergehenden Ansprüche, **dadurch gekennzeichnet, dass** an einem Maschinenelement, beispielsweise an einem um eine vertikale Maschinenachse umlaufenden Rotor mehrere Arbeitspositionen (6) zum Sterilisieren jeweils eines Behälters (1) vorgesehen sind, und dass an jeder Arbeitsposition (5) ein mit einer UV-Quelle (2) in Verbindung stehendes und in einen zu sterilisierenden Behälter (1) einführbares Lichtleiterelement (3) vorgesehen ist.

10. Vorrichtung nach Anspruch 9, **dadurch gekennzeichnet, dass** jede Arbeitsposition (6) eine eigene UV-Quelle (2) aufweist.

11. Vorrichtung nach Anspruch 9, **dadurch gekennzeichnet, dass** für sämtliche Arbeitspositionen (6) oder jeweils für Gruppen von mehreren Arbeitspositionen (6) eine gemeinsame UV-Quelle (2) am Maschinenelement vorgesehen ist.

12. Vorrichtung nach einem der vorhergehenden Ansprüche, **dadurch gekennzeichnet, dass** optische Element (4) derart ausgebildet ist, dass die Intensität der aus diesem Element austretenden Strahlung betrachtet über die Oberfläche des optischen Elementes (4) ungleichmäßig ist.

## Claims

1. Method for sterilising containers, where the inside surface of the container is treated with ultraviolet radiation from an ultraviolet source (2) that is emitted in the interior of the container, **characterised in that** the ultraviolet radiation from the ultraviolet source (2), which is situated completely outside the container (1), is emitted by means of an optical waveguide element (3) that is introduced through a container mouth (1.1) into the interior of the container.

2. Method according to claim 1, **characterised by** the use of a flexible optical waveguide element (3).

3. Method according to claim 1 or 2, **characterised by** the use of an optical waveguide element (3), which, at its end that is introduced into the respective container interior, is configured for emission of the ultraviolet radiation (5) over a three-dimensional aperture angle of 360° or approximately 360° in each spatial plane.

4. Method according to claim 1 or 2, **characterised by** the use of an optical waveguide element (3), which, at its end that is introduced into the respective container interior, is configured for a ring-shaped emission of the ultraviolet radiation (5) over a three-dimensional aperture angle of 360°, wherein the exit angle of the ultraviolet radiation (5) is modifiable in a controlled manner.

5. Apparatus for sterilising containers (1) with ultraviolet radiation, using an ultraviolet radiation source (2), the radiation of which is emitted in the interior of the container, **characterised in that** the output of the ultraviolet source (2) is connected to at least one optical waveguide element (3), which is introducible, with one end (3.1) that is remote from the ultraviolet source (2), through the container mouth (1.1) into the respective container for the emission of the ultraviolet radiation.

6. Apparatus according to claim 5, **characterised in that** the optical waveguide element (3) is configured at the end for emission of the ultraviolet radiation (5) over a three-dimensional aperture angle of 360° or almost 360° in each spatial plane.

7. Apparatus according to claim 5, **characterised in that** the optical waveguide element (3) is configured at the end for a radial emission of ultraviolet radiation (5) over a three-dimensional aperture angle of 360°, wherein there are disposed means that modify the exit angle of the radiation in a controlled manner.

8. Apparatus according to claim 5, 6 or 7, **characterised in that** the optical waveguide element (3) is flexible.

9. Apparatus according to one of the preceding claims, **characterised in that** on a machine element, for example on a rotor that rotates about a vertical machine axis, a plurality of work positions (6) are provided, each one sterilising one container (1), and **in that** at each work position (5) there is provided an optical waveguide element (3) that communicates with an ultraviolet source (2) and is introducible into a container (1) to be sterilised.

10. Apparatus according to claim 9, **characterised in that** each work position (6) includes its own ultraviolet source (2).

11. Apparatus according to claim 9, **characterised in that** a common ultraviolet source (2) is provided on the machine element for all of the work positions (6) or respectively for groups of several work positions (6).

12. Apparatus according to one of the preceding claims, **characterised in that** optical element (4) is configured in such a manner that, when viewed over the surface of the optical element (4), the intensity of the radiation emitted from the said element is irregular.

## Revendications

1. Procédé de stérilisation de récipients, dans lequel la surface interne de récipient est traitée avec un rayonnement ultraviolet d'une source de rayons ultraviolets (2) diffusé à l'intérieur du récipient, **caractérisé en ce que**,
le rayonnement ultraviolet de la source de rayons ultraviolets se trouvant entièrement à l'extérieur du récipient (1) est diffusé par un élément de guide de lumière (3) introduit par un orifice du récipient (1.1) dans l'espace interne de récipient.

2. Procédé selon la revendication 1, **caractérisé par** l'utilisation d'un élément de guide de lumière (3) flexible.

3. Procédé selon la revendication 1 ou 2, **caractérisé par** l'utilisation d'un élément de guide de lumière (3) qui est configuré, à son extrémité introduite dans l'espace interne respectif du récipient, en vue d'une diffusion du rayonnement ultraviolet (5) avec un angle d'ouverture spatial de 360° ou pratiquement 360° dans chaque plan spatial.

4. Procédé selon la revendication 1 ou 2, **caractérisé par** l'utilisation d'un élément de guide de lumière (3) qui est configuré, à son extrémité introduite dans l'espace interne respectif du récipient, en vue d'une diffusion annulaire du rayonnement ultraviolet (5) avec un angle d'ouverture spatial de 360°, l'angle de sortie du rayonnement ultraviolet étant modifiable de façon commandée.

5. Dispositif de stérilisation de récipients (1) avec rayonnement ultraviolet, avec une source de rayons ultraviolets (2) dont le rayonnement est diffusé à l'intérieur du récipient, **caractérisé en ce que** la sortie de la source de rayons ultraviolets (2) est reliée à au moins un élément de guide de lumière (3) qui peut être introduit avec une extrémité (3.1) éloignée de la source de rayons ultraviolets (2) à travers l'orifice de récipient (1.1) dans le récipient respectif pour la diffusion du rayonnement ultraviolet.

6. Dispositif selon la revendication 5, **caractérisé en ce que** l'élément de guide de lumière (3) est configuré à l'extrémité pour une diffusion du rayonnement ultraviolet (5) avec un angle d'ouverture spatial de 360° ou pratiquement 360° dans chaque plan spatial.

7. Dispositif selon la revendication 5, **caractérisé en ce que** l'élément de guide de lumière (3) est configuré à l'extrémité prévue pour une diffusion radiale du rayonnement ultraviolet (5) avec un angle d'ouverture spatial de 360°, des moyens étant disposés qui modifient de façon commandée l'angle de sortie du rayonnement.

8. Dispositif selon la revendication 5, 6 ou 7, **caractérisé en ce que** l'élément de guide de lumière (3) est flexible.

9. Dispositif selon une des revendications précédentes, **caractérisé en ce que** au niveau d'un élément mécanique, par exemple au niveau d'un rotor tournant autour d'un axe mécanique vertical, sont prévues plusieurs positions de travail (6) de stérilisation à chaque fois d'un récipient (1), et **en ce que** dans chaque position de travail est prévu un élément de guide de lumière (3) relié à une source de rayons ultraviolets (2) et pouvant être introduit dans un récipient (1) à stériliser.

10. Dispositif selon la revendication 9, **caractérisé en ce que** chaque position de travail (6) comprend une propre source de rayons ultraviolets (2).

11. Dispositif selon la revendication 9, **caractérisé en ce que** pour toutes les positions de travail (6) ou respectivement pour des groupes de plusieurs positions de travail (6) est prévue une source de rayons ultraviolets (2) commune sur l'élément mécanique.

12. Dispositif selon une des revendications précédentes, **caractérisé en ce que** l'élément optique (4) est configuré de sorte que l'intensité du rayonnement émise de cet élément est inégale sur la surface de l'élément optique (4)
